(19) Europäisches Patentamt / European Patent Office / Office européen des brevets

(11) **EP 4 380 555 B1**

(12) **EUROPEAN PATENT SPECIFICATION**

(45) Date of publication and mention
of the grant of the patent:
**25.03.2026 Bulletin 2026/13**

(51) International Patent Classification (IPC):
*A61K 31/16* (2006.01)      *A61K 31/164* (2006.01)
*A61K 31/198* (2006.01)      *A61K 31/7004* (2006.01)
*A61K 35/747* (2015.01)      *A61K 36/00* (2006.01)
*A61P 13/02* (2006.01)

(21) Application number: **22761275.1**

(52) Cooperative Patent Classification (CPC):
(C-Sets available)
**A61P 13/02; A61K 31/16; A61K 31/164;
A61K 31/198; A61K 31/7004; A61K 35/747**

(Cont.)

(22) Date of filing: **02.08.2022**

(86) International application number:
**PCT/IB2022/057154**

(87) International publication number:
**WO 2023/012657 (09.02.2023 Gazette 2023/06)**

(54) **COMPOSITION COMPRISING D-MANNOSE, N-ACETYLCYSTEINE AND PALMITOYLETHANOLAMIDE AND ITS USE FOR THE TREATMENT OF URIRNARY TRACT INFECTIONS**

ZUSAMMENSETZUNG ENTHALTEND MANNOSE, N-ACETYLCYSTEIN UND PALMITOYLETHANOLAMID UND DESSEN VERWENDUNG ZUR BEHANDLUNNG VON HARNWEGSINFEKTIONEN

COMPOSITION COMPRENANT DU MANNOSE, DU N-ACETYLCYSTEINE ET DU PALMITOYLÉTHANOLAMIDE ET SON UTILISATION POUR LE TRAITEMENT DES INFECTIONS DES VOIES URINAIRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.08.2021 IT 202100021134**

(43) Date of publication of application:
**12.06.2024 Bulletin 2024/24**

(73) Proprietor: **Agave S.r.l.**
**59100 Prato (PO) (IT)**

(72) Inventors:
• **BRANDANI, Daniele**
  **59100 Prato (PO) (IT)**
• **TISO, Domenico**
  **59100 Prato (PO) (IT)**

(74) Representative: **Villa, Livia**
**ADV IP S.r.l.**
**Corso di Porta Vittoria 29**
**20122 Milano (IT)**

(56) References cited:
**WO-A1-2015/173715**

• **MARK FELDMAN ET AL: "Antimicrobial potential of endocannabinoid and endocannabinoid-like compounds against methicillin-resistant Staphylococcus aureus", SCIENTIFIC REPORTS, vol. 8, no. 1, 1 December 2018 (2018-12-01), XP055670086, DOI: 10.1038/s41598-018-35793-7**
• **SOTO SARA M.: "Importance of Biofilms in Urinary Tract Infections: New Therapeutic Approaches", ADVANCES IN BIOLOGY, vol. 2014, 2 July 2014 (2014-07-02), pages 1 - 13, XP055903814, ISSN: 2356-6582, DOI: 10.1155/2014/543974**

EP 4 380 555 B1

**(Cont. next page)**

(52) Cooperative Patent Classification (CPC): (Cont.)

C-Sets
**A61K 31/16, A61K 2300/00;**
**A61K 31/164, A61K 2300/00;**
**A61K 31/198, A61K 2300/00;**
**A61K 31/7004, A61K 2300/00;**
**A61K 35/747, A61K 2300/00**

## Description

FIELD OF THE INVENTION

The present invention concerns a composition for the treatment of urinary infections and associated disorders selected from inflammations of the urinary tract, such as for example, cystitis and urethritis, and the symptoms thereof.

BACKGROUND ART

Urinary tract infections, or urinary infections, are very widespread, especially among women. The common symptoms of urinary tract infections are: urgent and frequent need to urinate, dysuria, and pain in the lower stomach. Urinary tract infections limit the activities of the patient, who sometimes even has to be admitted to hospital.

Urinary infections are caused by pathogens, mainly *E. coli*-type pathogens, and are therefore generally treated with the administration of antibiotics. Nevertheless, the side effects of antibiotics are known. Furthermore, bacteria have increasingly proved to be resistant to antibiotics and biofilm formation has been found to be the main reason for the failure of antibiotic treatment in urinary tract infections. This is because when the bacteria are not eliminated completely, those which remain are hidden in the epithelium of the bladder and can proliferate once again, causing relapses and recurrent urinary tract infections.

In WO2015173715A1, an association of polymeric proanthocyanidins (PACS), from 36 mg to 120 mg, for using against the urinary tract infections (UTI's) is disclosed, the association optionally further comprising one of the following phytotherapeutic extracts with diuretic-draining liquids activity: Betula alba, Orthosiphon stamineus, Solidago virgaurea, Agropyron repens, Hieracium pilosella, Asparagus officinalis, Zea mays, Taraxacum officinalis, or further comprising D-mannose.

Mark Feldman et al. 2018 "Antimicrobial potential of endocannabinoid and endocannabinoid-like compounds against methicillin-resistant Staphylococcus aureus" investigated the antimicrobial activity of the endocannabinoid (EC) anandamide (AEA) and the endocannabinoid-like (EC-like), arachidonoyl serine (AraS) against methicillin resistant *S. aureus* strains (MRSA). Due to their anti biofilm action these agents could also be a promising alternative to antibiotic therapeutics against biofilm-associated MRSA infections.

Sara Soto et al. 2014 "Importance of Biofilms in Urinary Tract Infections: New Therapeutic Approaches" is a review on how bacterial biofilms play an important role in urinary tract infections (UTIs), being responsible for persistence infections causing relapses and acute prostatitis, and on new therapeutic approaches including catheters coated with hydrogels or antibiotics, nanoparticles, iontophoresis, biofilm enzyme inhibitors, liposomes, bacterial interference, bacteriophages, quorum sensing inhibitors, low-energy surface acoustic waves, and antiadhesion agents. The authors concluded that new antimicrobial drugs that inhibit bacterial virulence and biofilm formation are needed. Therefore, it is becoming increasingly necessary to provide new remedies which are efficacious and have reduced side effects and, optionally, can increase the efficaciousness of commonly known remedies for the treatment of urinary infections.

SUMMARY OF THE INVENTION

Said object has been achieved by a composition comprising mannose, at least one endocannabinoid and N-acetylcysteine (NAC), as stated in Claim 1.

In another aspect, the present invention concerns the use of said composition for the treatment of urinary infections and disorders associated therewith, as defined in the claims, optionally in combination with an antibiotic treatment.

The characteristics and advantages of the present invention will be evident from the following detailed description, the working embodiments provided for illustrative, non-limiting purposes.

The invention is defined in the claims. Any subject-matter beyond the scope of the claims is not part of the invention and provided for comparison or reference purposes only.

Any reference to a non-patentable method of treatment of the human or animal body by therapy involving a certain compound or composition is to be understood as a reference to said compound or composition for use in said method of treatment.

BRIEF DESCRIPTION OF THE FIGURES

Figure 1: Results of the test carried out in Example 10 (MIC): 1 = Comp. 1; 2 = fosfomycin; 3 = ceftriaxone; 4 = D-mannose; 5 = medium (TBS/TSYEM) + Comp. 1; 6 = medium (TBS/TSYEM); 7 = control; A = *E. coli*; B = *E. faecium*; C

= *K. pneumoniae.*

Figure 2: Results of the test carried out in Example 11 (MBIC) with the composition (Comp. 1) according to preferred aspects of the invention - Observation under stereomicroscope of the biofilm of the various microorganisms: A = *E. Coli*; B = *E. faecium*; C = *K. pneumoniae.*

Figure 3: Results of the test carried out in Example 11 (MBIC) with *Enterococcus faecium* - Observation under optical microscope with enlargement factor of 400x: 1 = Comp. 1; 2 = fosfomycin.

Figure 4: Results of the test carried out in Example 11 (MBIC) with *E. coli* - Observation under optical microscope with enlargement factor of 400x: 1 = Comp. 1; 2 = fosfomycin

Figure 5: Results of the test carried out in Example 11 (MBIC) with *Klebsiella pneumoniae* - Observation under optical microscope with enlargement factor of 400x: 1 = Comp. 1; 2 = fosfomycin

Figure 6: Results of the test carried out in Example 11 (MBIC) with untreated controls - Observation under optical microscope with enlargement factor of 400x: A = *E. coli*; B = *E. faecium*; C= *K. pneumoniae*

Figure 7: Results of the test carried out in Example 12 (MBEC) with *Enterococcus faecium* - Observation under optical microscope with enlargement factor of 400x: 1 = Comp. 1; 2 = fosfomycin

Figure 8: Results of the test carried out in Example 12 (MBEC) with *E. coli* - Observation under optical microscope with enlargement factor of 400x: 1= Comp. 1; 2=fosfomycin

Figure 9: Results of the test carried out in Example 12 (MBEC) with *Klebsiella pneumoniae* - Observation under optical microscope with enlargement factor of 400x: 1 = Comp. 1; 2 = fosfomycin

Figure 10: Results of the cell viability assay carried out in Example 13; the viability values are expressed as percentage of the absorbency of the untreated cells.

Figure 11: Results of the treatment with different quantities of TNF-$\alpha$ according to Example 14.

Figure 12: Results of the test to determine anti-inflammatory activity of the product Comp. 1 according to Example 14.

Figure 13: Results of the test carried out in Example 15 (MBIC) with *E. coli* - Observation under optical microscope with enlargement factor of 400x: 1 = Comp. 1; 2 = D-Mannose

Figure 14: Results of the test carried out in Example 15 (MBIC) with *E. faecium*-Observation under optical microscope with enlargement factor of 400x: 1 = Comp. 1; 2 = D-Mannose

Figure 15: Results of the test carried out in Example 15 (MBIC) with *K. pneumoniae*-Observation under optical microscope with enlargement factor of 400x: 1= Comp. 1; 2 = D-Mannose

Figure 16: Results of the synergy test carried out in Example 16 (MBIC) with *E. coli*-Observation under optical microscope with enlargement factor of 400x: A: Comp. 1 + fosfomycin; B: Comp. 1 alone; 1 = fosfomycin concentrations; 2 = Comp. 1 concentrations.

Figure 17: schematic representation of the "chessboard analysis" to assess synergy in the MBIC test with *Escherichia coli*; a = fosfomycin concentrations; b = Comp. 1 concentrations. The growth of the biofilm is shown in greyscale (white = no biofilm); squares 1 and 2 indicate the wells in which a synergic effect of biofilm growth inhibition was observed. FIC index 1 = 0.75; FIC Index 2 = 0.56

Figure 18: Results of the synergy test carried out in Example 16 (MBIC) with *E. faecium* - Observation under optical microscope with enlargement factor of 400x: A: Comp. 1 + fosfomycin; B: Comp. 1 alone; 1 = fosfomycin concentrations; 2 = Comp. 1 concentrations.

Figure 19: schematic representation of the "chessboard analysis" to assess synergy in the MBIC test with *E. faecium*; a = fosfomycin concentrations; b = Comp. 1 concentrations. The growth of the biofilm is shown in greyscale (white = no biofilm); the squares 1, 2 and 3 indicate the wells in which a synergic effect of biofilm growth inhibition was observed. FIC Index 1 = 0.31; FIC Index 2 = 0.38; FIC Index 3 = 0.38.

Figure 20: *Enterococcus faecium* MBIC test, as per Example 17

Figure 21: *Klebsiella pneumoniae* MBIC test, as per Example 17

Figure 22: *Enterococcus faecium* MBEC test, as per Example 18

## DETAILED DESCRIPTION OF THE INVENTION

The invention therefore concerns a composition comprising D-mannose, N-acetylcysteine, and at least one endocannabinoid, said at least one endocannabinoid being palmitoylethanolamide (PEA).

Endocannabinoids have a lipid nature and derive from a polyunsaturated fatty acid, namely arachidonic acid. They are produced from biosynthetic precursors of a phospholipid kind and activate receptors of type 1 cannabinoids (CB1) - which are extremely abundant in the brain but also in peripheral tissues - and of type 2 cannabinoids (CB2), that are mainly expressed, meanwhile, in immune cells but are also expressed by glial cells within the central nervous system.

For the purposes of the present invention, the term "at least one endocannabinoid" means anandamide (or arachidonoyl ethanolamide, AEA), docosatetraenoylethanolamide (DEA), homo-linolenylethanolamide (HEA), 2-arachidonoylglycerol (2-AG), 2-arachidonyl-glyceryl-ether (noladin ether, 2-AGE), virodhamine, N-arachidonoyl-dopamine (NADA), palmitoylethanolamide (PEA), oleoylethanolamide (OEA), and their mixtures.

In the composition of the invention, said at least one endocannabinoid is palmitoylethanolamide (PEA).

PEA has, as its main action mechanism, an increase of CB2 receptors' expression, which is achieved through a genomic mechanism involving the activation of PPAR-$\alpha$. The stimulation of CB2 receptors promotes the release of endogenous opioids, with an analgesic effect, and the release of anti-inflammatory cytokines.

N-acetylcysteine (NAC) is N-acetylated cysteine. It is also known as $\alpha$-acetamido-$\beta$-mercaptopropionic acid.

Mannose is a constituent of numerous simple and complex polysaccharides. It constitutes, for example, the molecular base of mannans, reserve polysaccharides found in certain plant species (example, the palm) or, associated with galactose (mannogalactans).

The antibacterial action of mannose has been studied over recent years. Mannose can, in fact, interfere with the microbial adhesion of microbes known as 'mannose-sensitive' (or type 1 microbes).

The mannose according to the present invention is D-mannose.

Preferably, the mannose is pure D-mannose (i.e. a compound comprising at least 95% pure D-mannose, preferably at least 99% pure D-mannose, as measured by HPLC), or is a compound which can release D-mannose - upon ingestion - by enzymatic digestion.

In preferred embodiments of the invention, said mannose is in a weight ratio of 1:1 to 20:1 to said at least one endocannabinoid or said N-acetylcysteine; more preferably, in a weight ratio of 1:1 to 15:1, even more preferably 3:1 to 10:1.

Preferably, said at least one endocannabinoid and said N-acetylcysteine are in a weight ratio of 3:1 to 1:3, more preferably of 2:1 to 1:2, even more preferably about 1:1.

According to preferred embodiments, the composition according to the invention comprises up to 50 wt% mannose, more preferably up to 40 wt% mannose.

For the purposes of the present invention, unless otherwise specified, "wt%" means percentage by weight, based on the weight of the composition of the invention.

For the purposes of the present invention, unless otherwise specified, "comprises up to" with reference to a certain ingredient means that said ingredient is present in the composition, for example, in a minimum concentration of at least or equal to 0.001 wt%. In preferred aspects of the invention, the composition according to the invention comprises 20-40 wt% mannose, more preferably 30-40 wt% mannose, based on the total weight of the composition.

In preferred aspects of the invention, the composition according to the invention comprises 40-70 wt% mannose, based on the total weight of the active ingredients.

"Active ingredients" of the composition means the ingredients which perform a biological activity, including substances with a therapeutic effect.

According to preferred embodiments, the composition according to the invention comprises up to 15 wt% N-acetylcysteine, more preferably up to 10 wt% N-acetylcysteine.

In preferred aspects of the invention, the composition according to the invention comprises 5-10 wt% N-acetylcysteine, based on the total weight of the composition.

In preferred aspects of the invention, the composition according to the invention comprises 10-20 wt% N-acetylcysteine, based on the total weight of the active ingredients.

According to preferred embodiments, the composition according to the invention comprises up to 15 wt% of at least one endocannabinoid, more preferably up to 10 wt% of at least one endocannabinoid.

In preferred aspects of the invention, the composition according to the invention comprises 5-10 wt% of at least one endocannabinoid, based on the total weight of the composition.

In preferred aspects of the invention, the composition according to the invention comprises 10-20 wt% of at least one endocannabinoid, based on the total weight of the active ingredients.

In preferred embodiments, the composition according to the invention furthermore comprises lactobacilli.

Suitable lactobacilli for the composition of the present invention include, preferably but non exclusively, lactobacilli of the following species: L. *casei, L. paracasei, L. gasseri, L. plantarum, L. rhamnosus, L. acidophilus, L. crispatus.* Optionally, the composition can comprise a mixture of different lactobacilli.

Preferably, the composition according to the invention comprises lactobacilli of the species *Lactobacillus rhamnosus* (*L. rhamnosus*). Particularly preferred is LR04 strain *L. rhamnosus.*

In certain embodiments, said at least one lactobacillus is lyophilised. The process of lyophilisation allows to maintain viability of the bacteria, which are reactivated after rehydration, which occurs upon contact with the skin surface and/or the mucosa.

In particularly preferred embodiments, said at least one lactobacillus is tyndallised. "Tyndallised" means the lactobacillus has undergone a particular heat treatment inactiving the same, thus making it incapable of metabolising and reproducing. Lactobacilli are treated, together with their growth medium, which is constantly controlled and optimised, and also contains the substances produced by their metabolism. The lyophilisate thus obtained contains therefore both the probiotic cellular parts and the normal metabolism substances of the strains (vitamins, glycoproteins, micronutrients), and therefore can be as efficacious as the intake of the same living strains.

As the health of the urinary tract is based primarily on a condition of intestinal eubiosis, without wishing to be bound to any theory, it is believed that probiotics, in particular lactobacilli, can promote intestinal eubiosis and therefore prevent the colonisation of the urinary epithelium by harmful microorganisms.

Preferably, the composition according to the invention comprises at least $1\text{-}10 \times 10^7$ UFC (unit-forming colonies) of lactobacilli, more preferably at least $1\text{-}10 \times 10^8$ UFC of lactobacilli, even more preferably at least $1\text{-}10 \times 10^9$ UFC of lactobacilli.

In preferred embodiments, the composition according to the invention furthermore comprises hibiscus. "Hibiscus" means, preferably, a hibiscus extract obtained from the flowers of *Hibiscus sabdariffa,* according to commonly known methods. For example, the extract of hibiscus can be obtained by extraction in water of the active ingredients from the stems or petals of *Hibiscus sabdariffa.* The extract obtained can furthermore be concentrated or desiccated before being added to the composition according to the invention.

According to preferred embodiments of the composition according to the invention, said hibiscus is in a weight ratio of 3:1 to 1:3 to said at least one endocannabinoid or said N-acetylcysteine; more preferably in a weight ratio of 2:1 to 1:2, even more preferably 1.5:1 to 1:1.5.

According to preferred embodiments, the composition according to the invention comprises up to 20 wt% hibiscus, more preferably up to 10 wt% hibiscus.

In preferred aspects of the invention, the composition according to the invention comprises 2-10 wt% hibiscus, more preferably 3-6 % of hibiscus.

In preferred aspects of the invention, the composition according to the invention comprises 5-10 wt% hibiscus, based on the total weight of the active ingredients.

In preferred embodiments, the composition according to the invention is in the form of a unitary dose.

Preferably, said unitary dose comprises 100-1000 mg of said at least one endocannabinoid, more preferably 200-800 mg, even more preferably 300-600 mg.

Preferably, said unitary dose comprises 100-1000 mg N-acetylcysteine, more preferably 200-800 mg, even more preferably 300-600 mg.

Preferably, said unitary dose comprises 500-5000 mg mannose, more preferably 1000-4000 mg, even more preferably 1500-3000 mg.

Preferably, said unitary dose furthermore comprises 50-700 mg hibiscus, more preferably 100-500 mg, even more preferably 200-400 mg.

Preferably, said unitary dose furthermore comprises $1\text{-}10 \times 10^7$ UFC lactobacilli, more preferably $1\text{-}10 \times 10^8$ UFC, even more preferably $1\text{-}10 \times 10^9$ UFC.

The compositions, and unitary doses, comprising the following ingredients are furthermore preferred:

| | |
|---|---|
| - palmitoylethanolamide | 600 mg |
| - N-acetylcysteine | 600 mg |
| - D-mannose | 3000 mg |

or

| | |
|---|---|
| - palmitoylethanolamide | 300 mg |
| - N-acetylcysteine | 300 mg |
| - D-mannose | 1500 mg |

or

| | |
|---|---|
| - palmitoylethanolamide | 600 mg |
| - N-acetylcysteine | 600 mg |
| - D-mannose | 3000 mg |
| - hibiscus | 400 mg |

or

| | |
|---|---|
| - palmitoylethanolamide | 300 mg |
| - N-acetylcysteine | 300 mg |

(continued)

| | |
|---|---|
| - D-mannose | 1500 mg |
| - hibiscus | 200 mg |

or

| | |
|---|---|
| - palmitoylethanolamide | 600 mg |
| - N-acetylcysteine | 600 mg |
| - D-mannose | 3000 mg |
| - *L. rhamnosus* LR04 | 2x $10^9$ UFC |

or

| | |
|---|---|
| - palmitoylethanolamide | 300 mg |
| - N-acetylcysteine | 300 mg |
| - D-mannose | 1500 mg |
| - *L. rhamnosus* LR04 | 1x $10^9$ UFC |

or

| | |
|---|---|
| - palmitoylethanolamide | 600 mg |
| - N-acetylcysteine | 600 mg |
| - D-mannose | 3000 mg |
| - hibiscus | 400 mg |
| - *L. rhamnosus* LR04 | 2x $10^9$ UFC |

or

| | |
|---|---|
| - palmitoylethanolamide | 300 mg |
| - N-acetylcysteine | 300 mg |
| - D-mannose | 1500 mg |
| - hibiscus | 200 mg |
| - *L. rhamnosus* LR04 | 1x $10^9$ UFC |

It should be understood that the preferred aspects specified for the single components should likewise be considered preferred in the unitary dose embodiments described above.

The composition according to the invention, also in the form of unitary dose, can furthermore comprise pharmaceutically acceptable excipients. The word "excipient" means a compound or a mixture thereof suitable for use in the formulation of the composition. For example, an excipient for use in a pharmaceutical formulation generally must not cause an adverse response in a subject, nor should it significantly inhibit the efficaciousness of the composition.

Suitable excipients include acidifiers, acidity regulators, anti-caking agents, antioxidants, bulking agents, resistance agents, gelling agents, coating agents, modified starches, sequestering agents, thickeners, sweeteners, diluents, disaggregating agents, glidants, colorants, binders, lubricants, stabilisers, adsorbents, preservatives, humectants, flavourings, film-forming agents, emulsifiers, wetting agents, release retardants, and mixtures thereof.

Preferably, said excipients are potassium sorbate, sodium benzoate, ε-polylysine, sucralose, maltodextrin, citric acid, sodium carbonate, calcium carbonate, magnesium carbonate, magnesium stearate, stearic acid, polyethylene glycol, natural starch, partially hydrolysed starch, modified starch, lactose, calcium phosphate, calcium carbonate, calcium sulphate, polyvinylpyrrolidone, silica, colloidal silica, precipitated silica, magnesium silicate, aluminium silicates, sodium lauryl sulphate, magnesium lauryl sulphate, methacrylate copolymers, sodium dehydroacetate, xanthan gum, guar gum, tara gum, locust bean gum, fenugreek gum, Arabic gum, alginic acid, sodium alginate, propylene glycol alginate, sodium croscarmellose, polyvinylpolypyrrolidone, glyceryl behenate, titanium dioxide, indigo carmine, cellulose, modified cellulose, calcium carboxymethylcellulose, sodium carboxymethylcellulose, microcrystalline cellulose, ethylcellulose, gelatin, ethylcellulose, hydroxyethylcellulose, hydroxypropylcellulose, polydextrose, carrageenan, methylcellulose, saccharose, saccharose esters, sorbitol, xylitol, dextrose, fructose, maltitol, tragacanth gum, pectin, agar-agar, carboxypolymethylene, hydroxypropylmethylcellulose, tragacanth, mannitol, silicon dioxide, or their mixtures. Preferred excipients include an acidifier, such as citric acid, a sweetener, such as sucralose, an anti-caking agent, such as silicon dioxide.

In certain embodiments, the composition according to the invention consists essentially of mannose, N-acetylcysteine, and at least one endocannabinoid, optionally hibiscus and lactobacilli. The term "consists essentially of" means that mannose, N-acetylcysteine, and at least one endocannabinoid, optionally hibiscus and lactobacilli, are the sole active ingredients present in the composition, whereas any further components or excipients do not interfere with their action.

In preferred embodiments, the composition according to the invention consists of mannose, N-acetylcysteine, and at least one endocannabinoid, optionally hibiscus and/or lactobacilli and optionally pharmaceutically acceptable excipients.

It should be understood that all the aspects specified above as preferred and advantageous for the composition and the components thereof, are likewise also preferred and advantageous for these embodiments.

The composition of the present invention can be prepared applying commonly known methods. In fact, for oral administration, the components can, for example, be mixed as they are, or with one or more excipients, enclosed in soft-gel capsules or in a solid form, such as tablets, mini-tablets, micro-tablets, granules, micro-granules, pellets, multi particulate, micronised particulate, powder, or in the form of a solution, emulsion or gel, in a vial, or as drops, or a spray.

Preferably, the composition according to the invention is in the form of a granulate, more preferably to be taken dissolved in water.

Preferably, the composition according to the invention is formulated as a food supplement.

In another aspect, the present invention concerns the use of the composition described above for the treatment of urinary tract infections. Preferably, the present invention concerns the use of the composition described above for the treatment of urinary tract infections caused by mannose-sensitive bacteria.

For the purposes of the present invention, the term "treatment" is meant to include the administration of said composition to a subject suffering from a urinary infection or at risk of developing urinary infections, with the aim of improving the overall condition thereof, and likewise with the aim of slowing down, alleviating, reducing, and/or preventing the associated disorders and any alteration of said subject's bodily function. In particular, the treatment of urinary infections involves alleviating and/or preventing the disorders associated therewith, such as acute or chronic inflammation of the urinary tract, in particular cystitis and urethritis, and the symptoms thereof.

Therefore, the present invention also concerns the use of the composition described above for treatment of disorders associated with urinary tract infections.

The composition according to the invention has surprisingly allowed to treat urinary infections and the disorders associated therewith.

The activities of the composition according to the invention are the result of the synergic action of the components thereof.

In particular, the ingredients present in the composition according to the invention act synergically by:

- promoting complete emptying of the bladder and therefore elimination of the bacteria present therein, thanks to the diuretic and acidifying proprieties thereof;
- preventing the colonisation of the epithelium of the urinary tract, thanks to the capacity to bind the bacterial fimbriae, in particular those of mannose-sensitive bacteria, therefore inhibiting bacterial attachment;
- reducing the inflammation and algesia associated with urinary infections;
- antagonising the formation of bacterial biofilm and/or disaggregating the polymeric membrane of bacterial biofilms once already formed;
- promoting intestinal eubiosis, consequently preventing the colonisation of the epithelium of the urinary tract.

In particular, the composition according to the invention can act synergically within bacterial biofilm, inhibiting the formation thereof and reducing the biomass of biofilms which have already formed.

Furthermore, the composition according to the invention can boost the action of antibiotics in the treatment of urinary infections and associated disorders.

The composition according to the invention is preferably administered via oral, buccal, or sublingual route.

More preferably, the composition according to the invention is administered at least once a day. For the purposes of the present invention, the term "day" means a period of $24\pm4$ hours.

In preferred embodiments, the composition according to the invention is administered from one to three times per day in the form of a unitary dose, as described above.

Preferably the treatment lasts at least one week, for example, by administering the composition according to the invention once or twice per day in the form of a unitary dose for at least 7 days, then more preferably by administering the composition according to the invention once a day in the form of a unitary dose for a maintenance period of 7 to 30 days.

In preferred embodiments, the composition according to the invention is administered together with one or more antibiotics.

Preferably, the present invention therefore also concenrs a pharmaceutical product comprising the composition according to the invention and at least one antibiotic; furthermore, the present invention further concerns said pharmaceutical product and its use for the treatment of urinary infections and associated disorders.

To this end, said pharmaceutical product may be in the form of a kit for the simultaneous, separate, or sequential administration of the composition according to the invention and the at least one antibiotic.

Preferably, said at least one antibiotic is selected from the group comprising:

fosfomycin, ampicillin, amoxicillin, levofloxacin, ciprofloxacin, lomefloxacin, nitrofurantoin, sulfamethoxazole, trimethoprim, and their mixtures. The dosage of said at least one antibiotic is established by the clinician on an individual basis for each patient. It should be understood that are deemed to be described and therefore likewise preferred all the possible combinations of the preferred aspects of the components of the composition, as stated above.

It should furthermore be understood that all the aspects specified as preferred and advantageous for the composition and the components thereof, are likewise also deemed to be preferred and advantageous for the preparation and the uses of said composition. Below are working examples of the present invention provided for non-limiting, illustrative purposes.

## EXAMPLES

In Examples 1-10 below, compositions according to the present invention were prepared. The ingredients utilised in the preparation of the compositions were:

- Extract of dried Hibiscus flowers (*Hibiscus sabdariffa L.*): HiBCYN™, obtained from AKAY Flavours&Aromatics Pvt. Ltd, India, hereinafter shortly referred to as "Hibiscus"
- *Lactobacillus rhamnosus* LR04 (DSM16605), microencapsulated lyophilisate, obtained from Probiotical S.p.A., Italy (> 100 x $10^9$ cells/g)
- water-dispersible palmitoylethanolamide, obtained from Frau Pharma Srl, Italy
- N-acetyl -L-cysteine, crystalline powder, obtained from CJ Haide (Ningbo) Biotech Co., Ltd, China
- D-mannose, crystalline powder, obtained from Shanghai Freemen, China.

Example 1

The following composition was prepared:

| | |
|---|---|
| Palmitoylethanolamide | 600 mg |
| N-acetyl -L-cysteine | 600 mg |
| D-mannose | 3000 mg |

Example 2

The following composition was prepared:

| | |
|---|---|
| Palmitoylethanolamide | 300 mg |
| N-acetyl -L-cysteine | 300 mg |
| D-mannose | 1500 mg |

Example 3

The following composition was prepared:

| | |
|---|---|
| Palmitoylethanolamide | 600 mg |

(continued)

| N-acetyl -L-cysteine | 600 mg |
|---|---|
| D-mannose | 3000 mg |
| Hibiscus | 400 mg |

Example 4

The following composition was prepared:

| Palmitoylethanolamide | 300 mg |
|---|---|
| N-acetyl -L-cysteine | 300 mg |
| D-mannose | 1500 mg |
| Hibiscus | 200 mg |

Example 5

The following composition was prepared:

| Palmitoylethanolamide | 600 mg |
|---|---|
| N-acetyl -L-cysteine | 600 mg |
| D-mannose | 3000 mg |
| *L. rhamnosus* LR04 | $2 \times 10^9$ UFC |

Example 6

The following composition was prepared:

| Palmitoylethanolamide | 300 mg |
|---|---|
| N-acetyl -L-cysteine | 300 mg |
| D-mannose | 1500 mg |
| *L. rhamnosus* LR04 | $1 \times 10^9$ UFC |

Example 7

The following composition was prepared:

| Palmitoylethanolamide | 600 mg |
|---|---|
| N-acetyl -L-cysteine | 600 mg |
| D-mannose | 3000 mg |
| Hibiscus | 400 mg |
| *L. rhamnosus* LR04 | $2 \times 10^9$ UFC |

Example 8

The following composition was prepared:

| Palmitoylethanolamide | 300 mg |
| N-acetyl -L-cysteine | 300 mg |
| D-mannose | 1500 mg |
| Hibiscus | 200 mg |
| *L. rhamnosus* LR04 | 1 x 10$^9$ UFC |

Example 9

The following composition was prepared:

| Ingredient | Percentage in the formula (%) |
| --- | --- |
| D-mannose | 36.5854 |
| Maltodextrin | 33.4146 |
| N-acetylcysteine | 7.439 |
| Palmitoylethanolamide | 7.3171 |
| Aroma | 7.3171 |
| Hibiscus (*Hibiscus sabdariffa* L.), desiccated flowers | 4.878 |
| *L. rhamnosus* LR04 (DSM 16605) microencapsulated | 1.2195 |
| Citric acid | 1.2195 |
| Sucralose | 0.3659 |
| Silicon dioxide | 0.2439 |

Example 10

A food supplement (Comp. 1) was prepared comprising:

| Palmitoylethanolamide | 300 mg |
| N-acetyl -L-cysteine | 300 mg |
| D-mannose | 1500 mg |
| Hibiscus | 200 mg |

The product was dissolved in sterile TSB/TSYEM medium and then utilised to perform the following tests.

Example 11 - Antimicrobial activity (MIC)

The antimicrobial activity of the product Comp. 1 in Example 10 was tested *in vitro* according to method CLSI M07 "Methods for dilution Antimicrobial susceptibility tests for bacteria that grow aerobically".
For this purpose, the MIC (Minimum Inhibitory Concentration) values were determined with respect to the main microorganisms responsible for urinary tract infections.
More specifically, the microorganisms utilised were: *Escherichia coli* (DSM 1576), *Klebsiella pneumoniae* (DSM 30104), and *Enterococcus faecalis*(DSM 20477).
For each experiment conducted, the bacteria stated above were revived from a cell bank stored at -80°C. The following chart (Table 1) shows the growth conditions of the microorganisms.

Table 1

| | Inoculum UFC/mL | Medium | Growth conditions | Incubation time |
| --- | --- | --- | --- | --- |
| *Escherichia coli* | 4.38x10$^5$ | TSB | 37°C aerobic | 24 h |

(continued)

|  | Inoculum UFC/mL | Medium | Growth conditions | Incubation time |
|---|---|---|---|---|
| *Enterococcus faecium* | $5.00 \times 10^5$ | TSYEM | 37°C aerobic | 24 h |
| *Klebsiella pneumoniae* | $6.50 \times 10^5$ | TSYEM | 37°C aerobic | 24 h |

The assay to determine the MIC was conducted in 96-well plates testing 12 concentrations (serial dilutions) of each sample. 200 μL of the sample, prepared at a concentration of 2x with respect to the maximum concentration being tested, was dispensed into the first well and 100 μL medium was added to the subsequent wells, performing serial dilutions of 1:2 up to 12th well. Finally, 100 μL of each microorganism at a concentration of $10^5$ was inoculated into each well.

Two antibiotics were used as controls: Fosfomycin (Sigma) and Ceftriaxone (Sigma) at different final concentrations based on the microorganism utilised; both the antibiotics were dissolved in sterile water and then filtered with 0.22 μm filters.

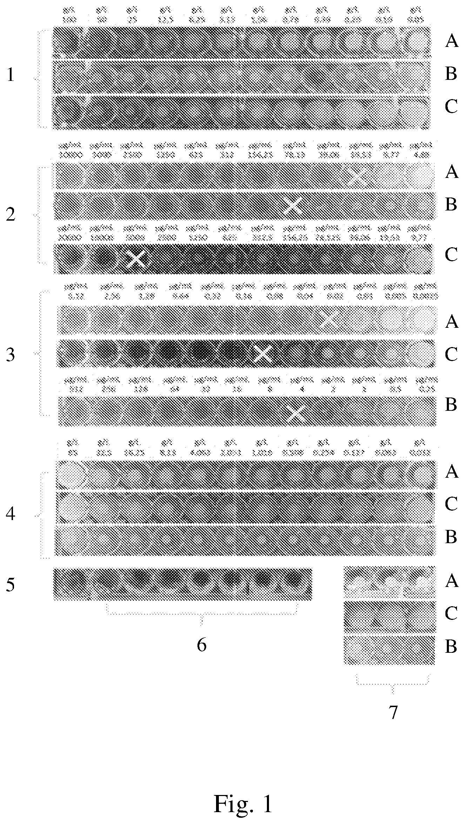

Growth was tested and the sterility of the medium and of the product was tested. The antimicrobial activity of the D-mannose was also assessed individually. The plates thus prepared were incubated at 37°C for 24h and the results were then observed (Figure 1). The final concentrations tested for each product analysed are stated in Table 2.

Table 2

|  | Comp. 1 g/L | Fosfomycin μg/mL | Ceftriaxone μg/mL | D-mannose g/L |
|---|---|---|---|---|
| *Escherichia coli* | 100-0.05 | 10000-4.8 | 5.12-0.0025 | 65-0.03 |
| *Enterococcus faecium* | 100-0.05 | 10000-4.8 | 512-0.25 | 65-0.03 |
| *Klebsiella pneumoniae* | 100-0.05 | 20000-9.7 | 5.12-0.0025 | 65-0.03 |

For fosfomycin, the MIC values were determined for each microorganism. *E. Coli* proved to be the most sensitive microorganism to this type of antibiotic, with an MIC value of 19.53 μg/mL. *Klebsiella,* on the contrary, proved to be the most resistant microorganism, with an MIC value of 5000 μg/mL. For *E. Faecium* the MIC value for fosfomycin was 78.13 μg/mL.

Antimicrobial activity was also assessed with D-mannose. As for the product Comp. 1, also the D-mannose alone in the range of concentrations tested did not demonstrate any antimicrobial activity, as cell growth was visible in all the wells.

Example 11 - Minimum biofilm inhibitory concentration (MBIC) - comparison with fosfomycin

The capacity of the product Comp. 1 in Example 10 and the fosfomycin to inhibit and to eradicate the formation of biofilm comprising the three target microorganisms was assessed by treating the microorganisms with 8 concentrations of the product, all lower than the MIC value determined in the previous test. The formation of the biofilm was viewed by means of *Crystal Violet* staining, wherein the stain penetrates the bacterial cells and turns them purple.

To determine the MBIC, 48-well plates were utilised. 200 μL of the sample, prepared at a concentration of 2x with respect to the maximum concentration being tested, was dispensed into the first well and 100 μL of medium was added to the subsequent wells, performing serial dilutions of 1:2 up to the 8th well. Finally, 100 μL of each microorganism at a concentration of $10^5$ were inoculated into each well. The biofilm inhibition activity of the fosfomycin, at a lower concentration with respect to the MIC value measured in Example 10, was also assessed and the sterility and growth tests were performed for each microorganism. The multiwell plates were incubated at 37°C for 24 hours and then the supernatant present was removed, together with the planktonic bacteria not attached to the plate.

The biofilm was then washed 3 times with PBS and subsequently the cells were fixed by means of incubation at 60°C for 2 hours, before being stained with crystal violet. The crystal violet staining was performed to assess the effect of the product and the fosfomycin on the formation of the biofilm. 50 μL crystal violet dissolved in deionised water was dispensed into each well and, after 5 minutes of incubation at room temperature, each well was finally washed three times with PBS 1x. The biofilm inhibition activity was viewed by microscopic observation under stereomicroscope and optical microscope using the phase-contrast method. The final concentrations tested for each sample are stated in Table 3.

Table 3

|  | Comp. 1 g/L | Fosfomycin μg/mL |
|---|---|---|
| *Escherichia coli* | 100-0.78 | 156.25-1.22 |

(continued)

|  | Comp. 1 g/L | Fosfomycin $\mu$g/mL |
|---|---|---|
| *Enterococcus faecium* | 100-0.78 | 321.5-2.51 |
| *Klebsiella pneumoniae* | 100-0.78 | 2500-19.53 |

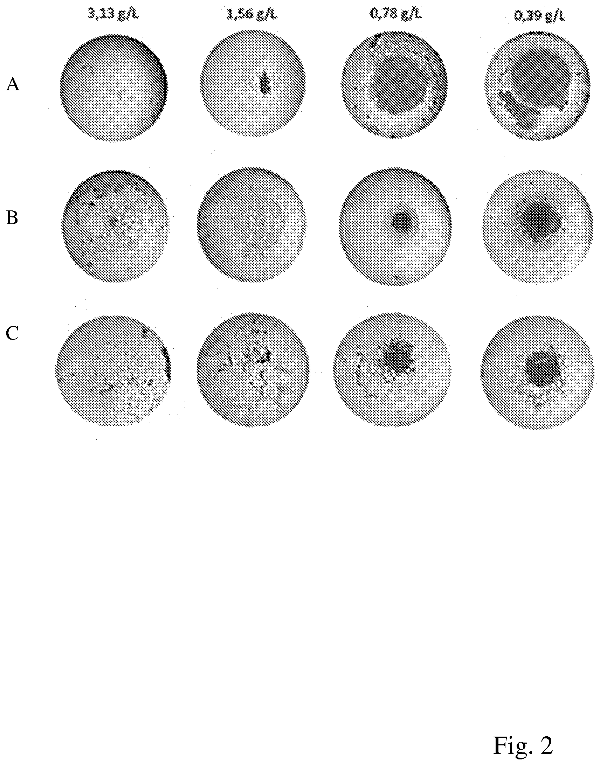

Upon observation of the biofilm under stereomicroscope (Figure 2) it emerged that the product Comp. 1 proved to have a biofilm formation inhibition activity.

As is clear from the staining, as the concentration of the product decreases, the biofilm increases. At a concentration of 1.56 g/L of the product Comp. 1 the reduction of the biofilm is already visible through macroscopic viewing.

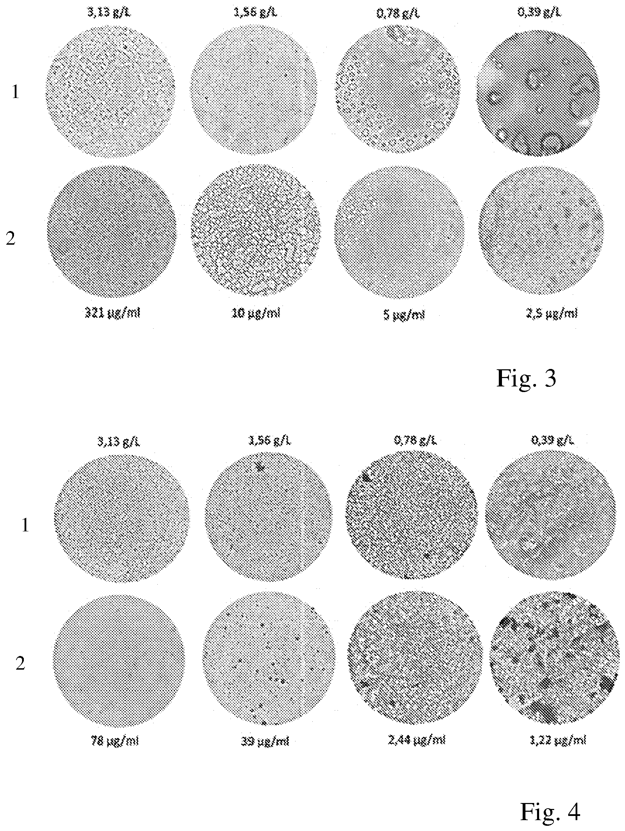

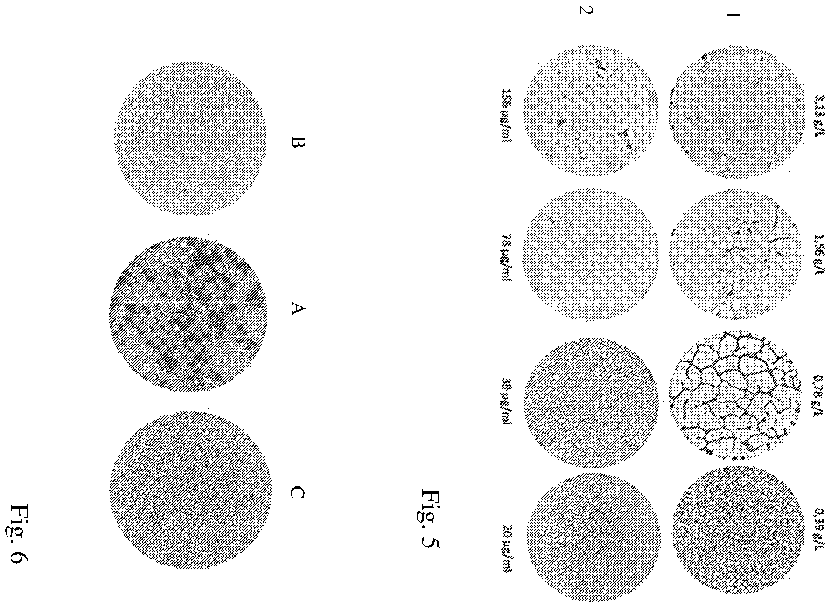

Subsequently photos were obtained under optical microscope with an enlargement factor of 400x to better assess the condition of the biofilm (Figures 3-6).

For all the microorganisms, at a product concentration of 1.56 g/L the biofilm was completely inhibited; only in the *E. coli* was there a very small number of attached cells present. At a concentration of 0.78 g/L, the biofilm inhibition effect was still very clear, especially on the *E. faecium* and on the *K. pneumoniae.*

On the *K. pneumoniae* and *E. coli,* fosfomycin had a biofilm inhibition effect at concentrations equal to or greater than 78 $\mu$g/mL, while on the *E. faecium* a concentration of at least 321 $\mu$g/mL was necessary.

Example 12- Minimum biofilm eradication concentration (MBEC)

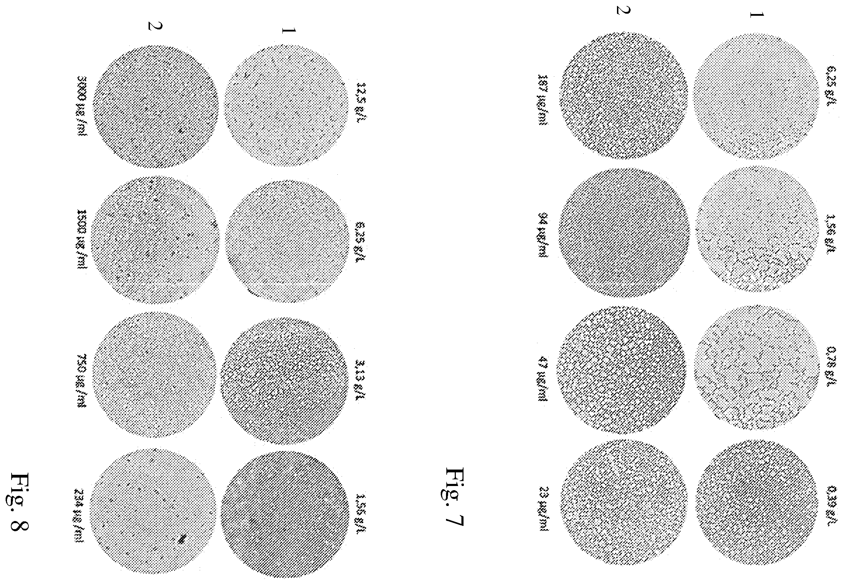

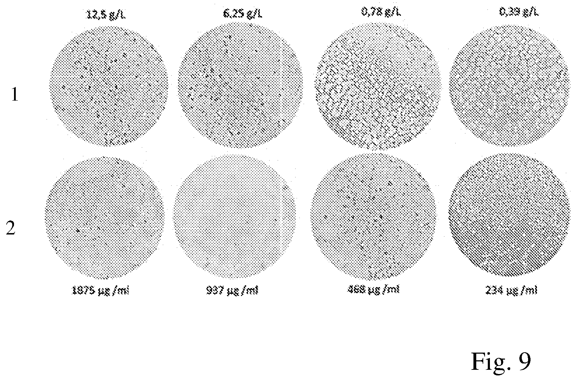

To determine the minimum biofilm eradication concentration, 48-well plates were utilised. The microorganisms were incubated for 72 h at 37°C to allow the formation of the biofilm. Once formed, the biofilm was treated with fosfomycin and with the product Comp. 1 in Example 10. In particular, after the formation of the biofilm, the supernatant was suctioned and replaced with solutions containing gradually decreasing concentrations of product or of fosfomycin. The plates thus prepared were incubated for 24 h at 37°C. At the end of the incubation time, the supernatant present in each well was removed, together with the bacteria that was not attached to the plate and, after washing, the plates were incubated at 60°C for 2 hours to fix the cells before the staining with crystal violet. The crystal violet staining was performed to assess the effect of the product and the fosfomycin on the removal of the biofilm. 50 $\mu$L crystal violet dissolved in deionised water was dispensed into each well. After 5 minutes of incubation at room temperature, each well was washed three times with PBS 1x. The biofilm eradication activity was viewed under microscopic observation (Figures 7-9).

The final concentrations tested for each product are stated in Table 4

Table 4

|  | Comp. 1 g/L | Fosfomycin $\mu$g/mL |
|---|---|---|
| *Escherichia coli* | 50-0.39 | 30000-234.38 |
| *Enterococcus faecium* | 50-0.39 | 3000-23.44 |
| *Klebsiella pneumoniae* | 50-0.39 | 3000-23.44 |

The product Comp. 1 had an eradication action on the biofilm formed of *K. Pneumoniae* and *E. faecium* at a concentration of 0.78 g/L and on *E.coli* at a concentration of 6.25g/L. The fosfomycin did not seem to have a biofilm eradication effect at either of the concentrations tested for *E. faecium* and *E.coli* while it had an activity on *K. pneumoniae* at concentrations greater than 468 $\mu$g/mL. The food supplement according to the invention acts, therefore, to both inhibit and eradicate the biofilm formed of the microorganisms *K. Pneumoniae, E. faecium* and *E. coli.* In the in vitro tests, the activity of the product is already significant at a concentration of 0.78 g/L.

Example 13 - Cytotoxic activity

We assessed the effect of the product Comp. 1 in Example 10 on the viability of a cell line derived from human bladder (HT1376) exploiting the redox reactions of the compound 3-(4.5-dimethylthiazol-2-the)-2.5-diphenyltetrazolium bromide (MTT), which forms purple crystals in the presence of viable cells.

The HT1376 cells, at a confluence of > 90%, were collected and diluted at a concentration of $10^5$cells/mL. Where not specified, the cells were cultured at 37°C and 5% $CO_2$ in complete *Eagle's Minimum Essential Medium* (EMEM with 1% penicillin/streptomycin, 1% non-essential amino acids, 2 mM L-glutamine), with supplemented with 10% of foetal bovine serum (FBS). 100 $\mu$L of this dilution ($10^4$ cells) were aliquoted into each well of a sterile 96-well plate, which was incubated overnight at 37°C and 5% $CO_2$. At the end of the incubation, the medium was removed and replaced with 100 $\mu$L of

complete EMEM supplemented with 1% FBS and containing the product Comp. 1 at the following concentrations (in mg/mL): 5 - 2.5 - 1.25 - 0.625 - 0.313 - 0.156 - 0.078 - 0.039 - 0.02 - 0.01 - 0.005 - 0.0024. Sodium dodecyl sulphate (SDS) was utilised as a positive control at a concentration of 1 mg/mL. The cells were incubated for 24 hours at 37°C and 5% $CO_2$. At the end of the treatment, the MTT was added to each well at the final concentration of 0.5 mg/mL, and incubation was continued for another two hours. The growth medium was then removed from the wells, and the reduced MTT crystals were solubilised with the addition of 100 μL of dimethyl sulfoxide (DMSO). After 30 minutes of incubation at room temperature, the absorbency at 595 nm of each sample was measured with an Infinite M NANO+ (Tecan) plate reader. Cell viability is expressed as percentage of the absorbency at 595 nm of the untreated cells (NT).

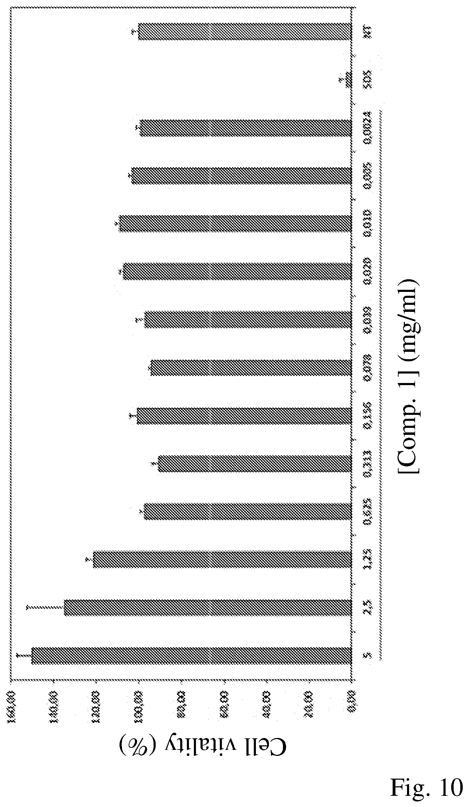

As shown in Figure 10, the product did not demonstrate a toxic effect on cell viability.

Example 14 - Anti-inflammatory activity

We assessed the capacity of the product Comp. 1 in Example 10 to inhibit or reduce the inflammatory state induced, in the same cells as in the previous example, by pro-inflammatory cytokine TNFα. This treatment led to the production of interleukin 8 (IL-8), which is cause of the "recruitment" of white blood cells to the site of inflammation. The IL-8 levels are directly proportional to the intensity of said inflammation.

The 6 product concentrations utilised in the anti-inflammatory test were chosen based on the cell viability test results. In particular, six concentrations of the product were utilised, decreasing from 0.625 mg/mL, as at concentrations greater than 0.625 mg/mL, the data from Example 13 showed an increase in cell viability. This is because the product per se leads to the reduction, and therefore the staining, of the MTT.

We first determined the optimal amount of TNFα to perform the test. The HT1376 cells, at a confluence of > 90%, were collected and diluted at a concentration of $10^4$ cells/mL. 1 mL of this dilution ($10^4$ cells) was aliquoted into each well of a sterile 48-well plate, which was incubated at 37°C and 5% $CO_2$ for 48h. At the end of the incubation, the medium was removed and replaced with 1 mL complete EMEM without FBS, containing TNFα at the following concentrations (in μg/mL): 0.2 - 0.1 - 0.05 - 0.025. The treatment was performed at 37°C and 5% $CO_2$ for a total of 6 hours; aliquots of 200 μL of the growth medium were taken at the start of the treatment (T0) and after 1, 2, 4 and 6 hours, and then analysed by ELISA test. For the anti-inflammatory activity test, the HT1376 cells, at a confluence of > 90%, were collected and diluted at a concentration of $10^4$cells/mL. 1 mL of this dilution ($10^4$ cells) was aliquoted into each well of a sterile 48-well plate, which was incubated at 37°C and 5% $CO_2$ for 48h. At the end of the incubation, the medium was removed and replaced with 500 μL of EMEM complete without FBS, containing 0.2 μg/mL of TNFα alone or with the product Comp. 1 at the following concentrations (in mg/mL): 0.625 - 0.313 - 0.156 - 0.078 - 0.039 - 0.02.

Anti-inflammatory drug dexamethasone (dex) at a concentration of 0.1 mg/mL was utilised as a positive control. The treatment was performed for 6 hours, at the end of which as aliquots of 200 μL of the growth medium were taken and then analysed by ELISA test. IL-8 measurement was performed on 100 μL of each aliquot kept, using the "Human IL-8 Uncoated ELISA Kit" (Invitrogen), according to the protocol supplied by the manufacturer. The quantification was performed by setting up a calibration curve with different concentrations of a standard IL-8 solution, supplied with the kit.

The anti-inflammatory effect is expressed as a variation percentage of the concentration of IL-8 with respect to the cells treated solely with TNFα.

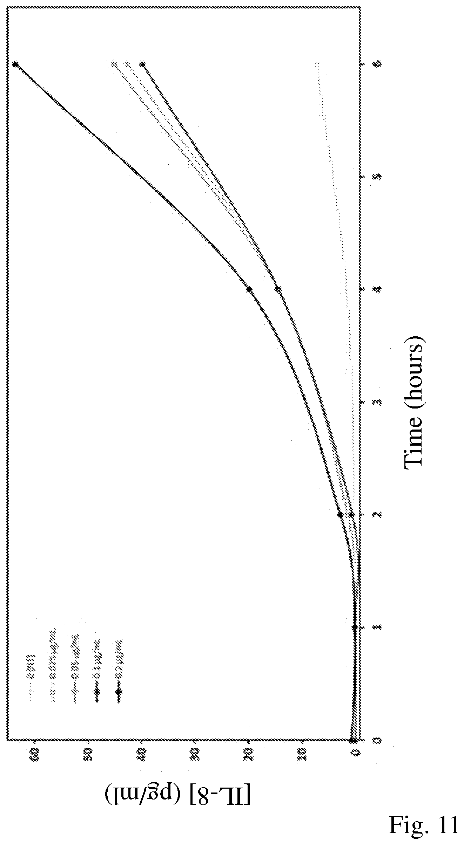

As can be seen (Figure 11), treatment with any amount of TNFα causes an overexpression of IL-8, with respect to the untreated cells (NT). In general, it seems that it takes at least 4 hours of treatment in order to see appreciable differences in the production of IL-8, but the greatest effect can be seen after 6 hours. The treatment with 0.025 - 0.05 or 0.1 μg/mL of TNFα seems to give result dare results, with a production of 40 - 45 μg/mL of IL-8 after 6 hours of treatment.

Increasing the TNFα to 0.2 μg/mL results in a greater production of interleukin, with a maximum of approximately 63.5 pg/mL after 6 hours of treatment.

In order to appreciate, with the greatest precision possible, the variations in the production of IL-8 due to treatment with the product, on the basis of this data, we therefore decided to treat the cells with 0.2 μg/mL TNFα for 6 hours.

The anti-inflammatory effect of the product was assessed by measuring the ability thereof to reduce the amount of IL-8 produced by the HT1376 cells following treatment with TNFα.

In particular, the cells were treated with pro-inflammatory cytokine TNFα in the conditions developed previously, with the product either absent or present, and then the amount of IL8 expressed was measured. Anti-inflammatory drug dexamethasone at a concentration of 0.1 mg/mL was utilised as a positive control.

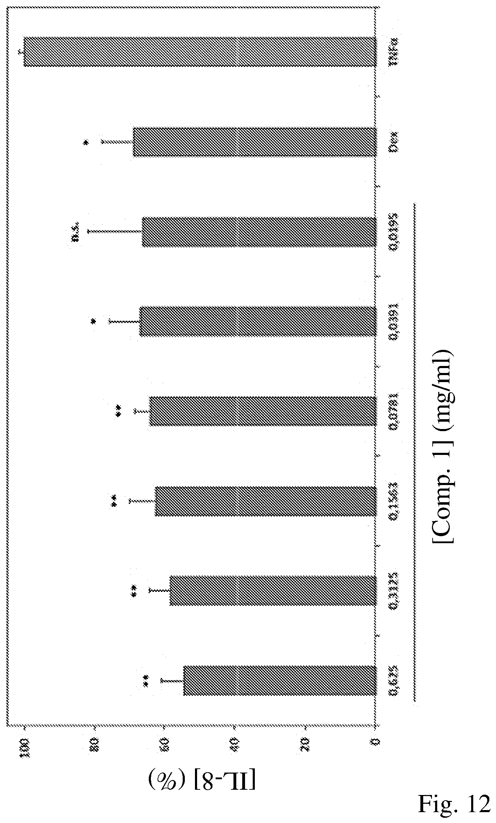

As shown in Figure 12 and Table 5, the product can reduce the overexpression of IL-8 induced by TNFα, in a dose-dependent manner, up to a concentration of 0.04 mg/mL: at this value, the IL-8 produced is approximately 33% less than that produced by the cells treated solely with TNFα, which is an effect that is comparable to that obtained with the Dexamethasone at a concentration of 0.1 mg/mL (31%).

The greatest effect is obtained with a product concentration of 0.625 mg/mL, with which a 45.5 %reduction in the production of IL-8 is observed.

Table 5

| Controls | | Product (mg/mL) | | | | | |
|---|---|---|---|---|---|---|---|
| TNF$\alpha$ (0.2 $\mu$g/mL) | 100 | 0.625 | 0.313 | 0.156 | 0.078 | 0.039 | 0.02 |
| Dex (0.1 mg/mL) | 69 | 54,5 | 58,4 | 62,6 | 64,2 | 66,9 | 66,4 |

Based on the data obtained, we can therefore state that the product has an inhibitory effect on the inflammation mediated by TNF$\alpha$.

Example 15 - Minimum biofilm inhibitory concentration (MBIC) - comparison with D-mannose

To determine the MBIC with the product Comp. 1 in Example 10 with respect to the D-mannose, 48-well plates were utilised. 200 $\mu$L of the product Comp. 1, prepared at a concentration of 2x with respect to the maximum concentration being tested, was dispensed into the first well and 200 $\mu$L of medium was added to the subsequent wells, performing serial dilutions of 1:2 up to the 8th well. Finally, 200 $\mu$L of each microorganism, at a concentration of $10^5$, was inoculated into each well. The biofilm inhibition activity of the D-mannose at the same concentration as that present in the product Comp. 1 was also assessed.

The multiwell plates were incubated at 37°C for 24 hours and then the supernatant present was removed, together with the planktonic bacteria not attached to the plate.

The biofilm was then washed 3 times with PBS and subsequently the cells were fixed by means of incubation at 60°C for 2 hours, before being stained with crystal violet. 100 $\mu$L crystal violet dissolved in deionised water was dispensed into each well and, after 5 minutes of incubation at room temperature, each well was finally washed three times with PBS 1x. The biofilm inhibition activity was viewed by microscopic observation under stereomicroscope and optical microscope using the phase-contrast method. The final concentrations tested for each compound are stated in Table 6.

Table 6

| | D-mannose (g/L) | Product in its entirety (g/L) |
|---|---|---|
| *Escherichia coli* | 65-0.51 | 100-0.78 |
| *Enterococcus faecium* | 65-0.51 | 100-0.78 |
| *Klebsiella pneumoniae* | 65-0.51 | 100-0.78 |

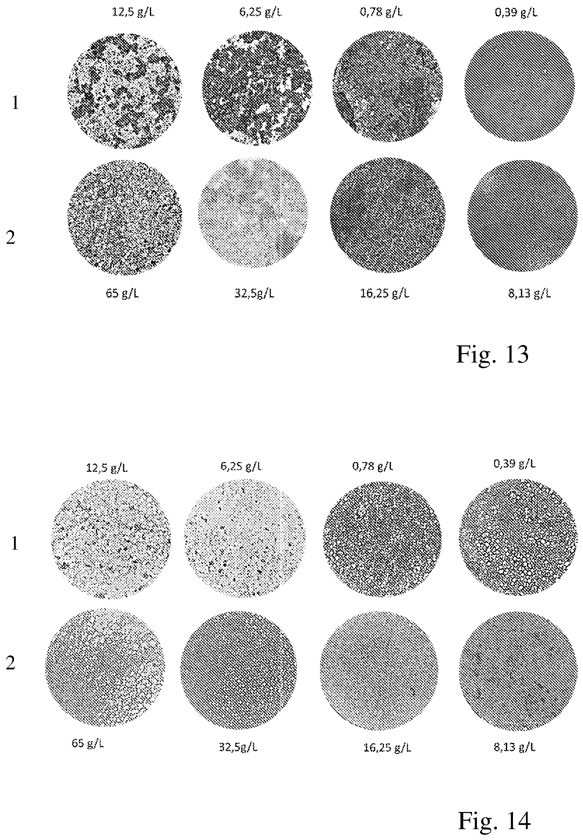

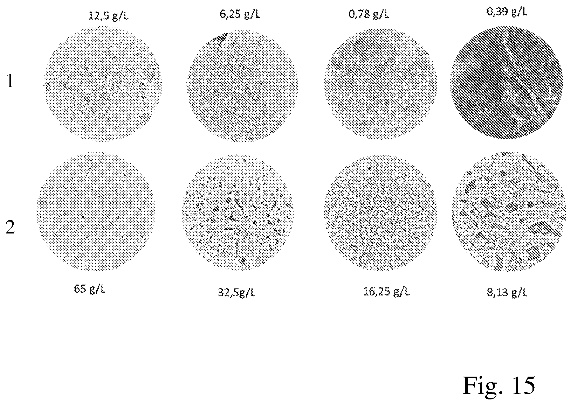

As shown in Figures 13, 14, 15, the D-mannose proved to have a biofilm formation inhibition activity.

The D-mannose proved to be active on *K. pneumoniae* at a concentration of 8.13 g/L and on *E. coli* at a concentration of 32.5 g/L. Said D-mannose has an evident activity on the biofilm comprising *E. faecium* solely at a concentration of 65 g/L.

Clearly, the product Comp. 1 according to preferred embodiments of the present invention proved to have a greater activity with respect to the D-mannose utilised alone and at the same concentration as that contained in the product Comp. 1.

Surprisingly, the results obtained from the minimum biofilm inhibitory concentration (MBIC) test show that to obtain the same efficaciousness as that obtained with the present invention on the *E. coli* biofilm, the concentration of the mannose, utilised alone and at the same concentration as that contained in the product Comp. 1, needed to be approximately 2.5 times greater. Surprisingly, the results obtained from the MBIC test show that, to obtain the same efficaciousness as obtained with the present invention on the biofilm comprising *K pneumoniae* and *E. faecium*, the concentration of the mannose, utilised alone and at the same concentration as that contained in the product Comp. 1, needed to be approximately 10 times greater.

Example 16 - Minimum biofilm inhibitory concentration (MBIC) - synergic activity with fosfomycin

This test was carried out according to the protocol known as at the "*broth microdilution checkerboard method*", which allows the inhibitory effect of two agents acting contemporaneously, by crossing serial dilutions thereof. In particular, 8 concentrations of the product Comp. 1 in Example 10 and 6 concentrations of fosfomycin were crossed. 48-well plates were utilised.

200 $\mu$L of the product Comp. 1, prepared at a concentration of 2x with respect to the maximum concentration being tested, was dispensed into the eight column and 200 $\mu$L of the medium was added to the subsequent wells, performing serial dilutions of 1:2 up to the second well on each row. Subsequently, 200 $\mu$L of fosfomycin, at a concentration 2x with respect to the MIC value (different for each microorganism), was dispensed into the first row and serials dilutions at 1:2 were performed on the columns up to penultimate row.

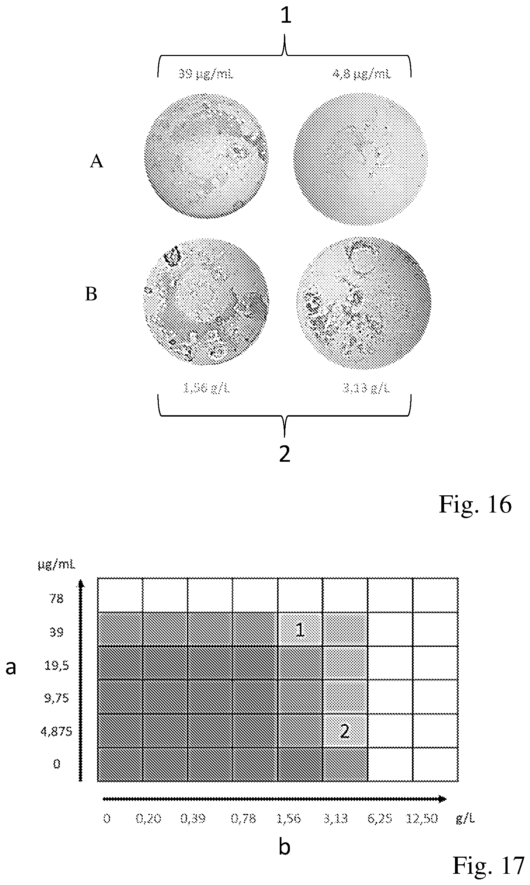

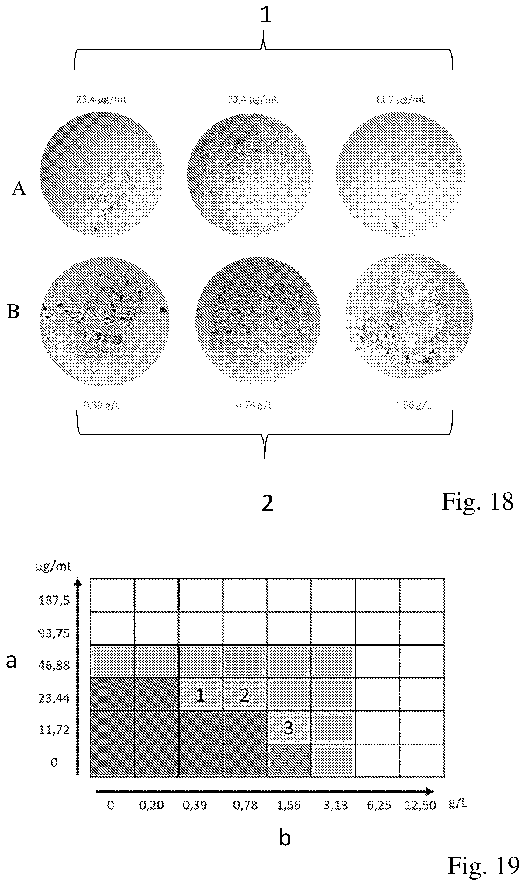

Finally, 200 μL of each microorganism, at a concentration of $10^5$, was inoculated into each well. Figures 17 and 19 provide graphical representations of the matrix of the concentrations performed to determine the anti-biofilm synergism, which highlight the combinations of compounds considered for the synergic effect.

The multiwell plates were incubated at 37°C for 24 hours and then the supernatant present was removed, together with the planktonic bacteria not attached to the plate. The biofilm was then washed 3 times with PBS and subsequently the cells were fixed by means of incubation at 60°C for 2 hours, before being stained with crystal violet. The crystal violet staining was performed to assess the effect of the product and the D-mannose on the formation of the biofilm. 100 μL of crystal violet dissolved in deionised water was dispensed into each well and, after 5 minutes of incubation at room temperature, each well was finally washed three times with PBS 1x. The biofilm inhibition activity was viewed by microscopic observation under stereomicroscope and optical microscope using the phase-contrast method.

Following incubation, the results obtained were analysed through crystal violet staining. Photos were obtained under optical microscope with an enlargement factor of 400x in order to better assess the condition of the biofilm.

Figures 16 and 18 show, for comparison purposes, the condition of the biofilm treated with the product alone (B) and at the same concentration combined with fosfomycin (A).

The data collected shows that, in certain combinations of Comp. 1/fosfomycin, the biofilm formed of the microorganisms *Escherichia coli* and *Enterococcus faecium* was reduced with respect to the corresponding condition with the products alone at the same concentration (Figures 16-19).

For these combinations, the FIC indexes were therefore calculated according to the following formula:

$$\text{FIC index} = \frac{\text{MBIC fosfomycin in association}}{\text{MBIC fosfomycin}} + \frac{\text{MBIC product in its entirety, in association}}{\text{MBIC product in its entirety}}$$

The association results to be synergic, if the FIC index values are < 1. When the FIC index has the value = 1 the compounds are not interacting reciprocally, whereas if the value is > 1, the combination of the compounds results to be antagonistic.

For both the *Escherichia coli* microorganism and the *Enterococcus faecium* microorganism, a synergic effect of combinations of the product Comp. 1 and fosfomycin on biofilm formation inhibition was observed.

Example 17 - Minimum biofilm inhibitory concentration (MBIC) - synergic anti-biofilm activity of the composition according to the invention

The composition of Example 1 was tested to assess the capacity to inhibit the formation of biofilm shown by the following microorganisms:

- *Klebsiella Pneumoniae* DSM 30104
- *Enterococcus Faecium* DSM 20477

The bacteria stated above were revived from the working cell bank, stored at -80°C, for each experiment conducted. The following table shows the growth conditions of the microorganisms:

|  | Inoculum (UFC/mL) | Medium | Growth conditions | Incubation time |
|---|---|---|---|---|
| *Klebsiella pneumoniae* | $10^5$ | TSYEM | 37°C aerobic | 24 h |
| *Enterococcus faecium* | $10^5$ | TSYEM | 37°C aerobic | 24 h |

The tests were conducted on the following formulations:

- Mannose 3000 mg + NAC 600 mg + PEA 600 mg (i.e. composition Example 1)
- Mannose 3000 mg + NAC 600 mg
- PEA 600 mg

The three formulations were dissolved at 100 gr/l in sterile TSB/TSYEM medium and then utilised to perform the MBIC test as follows.

MBIC 48-well plates were utilised for the determination. 800 μL sample, prepared at a concentration of 2x with respect to the maximum concentration being tested, was dispensed into the first well and 400 μL medium was added to the subsequent wells, performing serial dilutions of 1:2 up to the 8th well. Finally, 400 μL of each microorganism at a concentration of 10^5 was inoculated into each well. The multiwell plates were incubated at 37°C for 24 hours and then the supernatant present was removed, together with the planktonic bacteria not attached to the plate.

The biofilm was then washed 3 times with sterile deionised water and subsequently the cells were fixed by means of incubation at 60°C for 2 hours, before being stained with crystal violet. The crystal violet staining was performed to assess the effect of the product and the components thereof on the biofilm formation. 200 μL of 0.02% crystal violet dissolve in deionised water was dispensed into each well and, after 5 minutes of incubation at room temperature, each well was finally washed three times with sterile deionised water. The biofilm inhibition activity was viewed by microscopic observation under stereomicroscope.

At the end of the microscopic observation of the condition of the biofilm by staining, the stain fixed to the biofilm treated was extracted utilising 100% methanol for 5 minutes at room temperature. The methanolic extract thus obtained was analysed under spectrophotometer to measure the absorbency at 595 nm. As the solution stain intensity obtained is proportional to the biofilm remaining, absorbency is a quantitative parameter which can be used to measure the amount of biofilm. Absorbency was therefore utilised to determine the biofilm decrease percentage by comparing the data obtained following treatments against the untreated biofilm control.

*Results*

*- Enterococcus faecium*

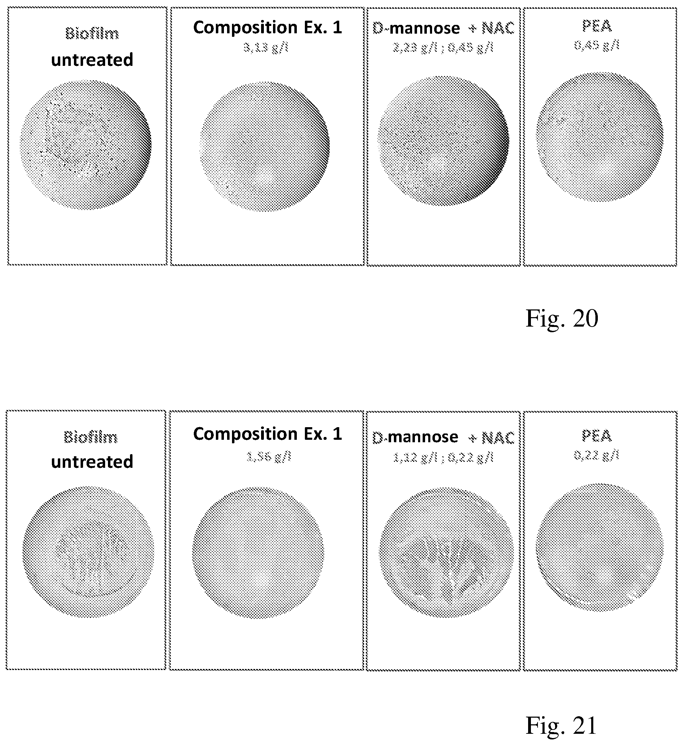

At the concentration of 3.13 g/L product of Example 1, an increased reduction in the biofilm can be acknowledged with respect to the treatment with D-mannose + NAC at the same concentration, as in the composition according to the invention (Figure 20).

Table 7 shows the percentage decrease of the treated biofilm compared to the untreated biofilm, in this case a synergy action of the composition of Example 1 is observed with respect to the individual components (Data highlighted).

Table 7

| *E. faecium* MBIC | Abs 595 nm | % decrease |
|---|---|---|
| Untreated biofilm | 0.318 | - |
| D-mannose + NAC + PEA | 0.241 | **24%** |
| D-mannose + NAC | 0.279 | 12% |
| PEA | 0.304 | 4% |

*- Klebsiella pneumoniae*

At the concentration of 1.56 g/L product of Example 1, a macroscopical view of the reduction in the biofilm can be acknowledged with respect to the treatment with D-mannose + NAC.

At the 1.56 g/L product concentration, an increased reduction in the biofilm can be acknowledged with respect to the treatment with D-mannose + NAC at the same concentration, as in the composition of Example 1 (Figure 21).

Table 8 shows the percentage decrease of the treated biofilm compared to the untreated biofilm, in this case, as with the previous microorganism, a synergy action of the composition of Example 1 is observed with respect to the individual components (Data highlighted).

Table 8

| MBIC *K. pneumoniae* | Abs 595 nm | % decrease |
|---|---|---|
| Untreated biofilm | 0.176 | - |
| D-mannose + NAC + PEA | 0.063 | **64.20%** |
| D-mannose + NAC | 0.201 | 0% |
| PEA | 0.129 | 26.70% |

From the data stated above for the MBIC test, it emerges that there was an evident synergy in the biofilm inhibition activity given by the composition according to the invention, compared to the individual components, in both *Klebsiella*

*pneumoniae* and *Enterococcus faecium.* Indeed, the sum of the reduction percentage achieved with the individual treatments was lower than the biofilm reduction percentage achieved with the composition according to the invention.

Example 18 - Minimum biofilm inhibitory concentration (MBIC) - synergic anti-biofilm activity of the composition according to the invention

As *Enterococcus Faecium* is held to be the most representative microorganism in urinary infections, for the purposes of the present study, the composition of Example 1 was also tested to assess the capacity thereof to eradicate the formation of biofilm formed thereby. To determine the minimum biofilm eradication concentration, 48-well plates were utilised. The microorganisms were incubated for 24 h at 37°C to allow the formation of the biofilm. Once formed, the biofilms were treated with the composition in Example 1 and the respective components (as per Example 17) to eradicate the biofilm. In particular, after the formation of the biofilm, the supernatant was suctioned and replaced with solutions containing decreasing concentrations of the product. The plates thus prepared were incubated for 24 h at 37°C. At the end of the incubation time, the supernatant present in each well was removed, together with the bacteria that was not attached to the plate and following the washes with sterile deionised water the plates were incubated at 60°C for 2 hours to fix the cells before the staining with crystal violet. The crystal violet staining was performed to assess the effect of the product and of the components thereof on the removal of the biofilm. 200 μL of crystal violet dissolved in deionised water at a concentration of 0.02% was dispensed into each well and, after 5 minutes of incubation at room temperature, each well was washed three times with sterile deionised water. The biofilm eradication activity was viewed under microscopic observation.

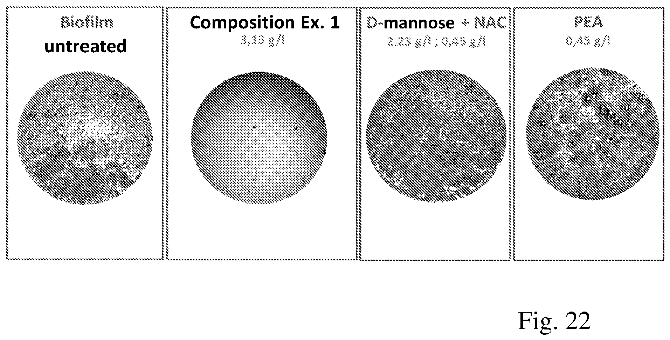

Figure 22 and Table 9 show the eradication test results the biofilm formed of *Enterococcus Faecium.*

Table 9

| MBEC *E. faecium* | Abs 595 nm | % decrease |
|---|---|---|
| Untreated biofilm | 2.09 | - |
| D-mannose + NAC + PEA | 1.581 | **24%** |
| D-mannose + NAC | 2.022 | 3% |
| PEA | 2.098 | 0% |

From the data stated above for the MBEC test, it emerges that there was an evident synergy in the *Enterococcus faecium* biofilm eradication activity given by the composition according to the invention, compared to the individual components. Indeed, the sum of the reduction percentage achieved with the individual treatments was lower than the biofilm reduction percentage achieved with the composition according to the invention.

**Claims**

1.  Composition comprising D-mannose, N-acetylcysteine, and at least one endocannabinoid, said at least one endocannabinoid being palmitoylethanolamide.

2.  The composition of claim 1, wherein said D-mannose is in a weight ratio of 1:1 to 20:1 with respect to said at least one endocannabinoid or to said N-acetylcysteine.

3.  The composition of claim 2, wherein said mannose is in a weight ratio of 1:1 to 15:1 with respect to said at least one endocannabinoid or to said N-acetylcysteine.

4.  The composition of claim 3, wherein said D-mannose is in a weight ratio of 3:1 to 10:1 with respect to said at least one endocannabinoid or to said N-acetylcysteine.

5.  The composition of any one claims 1-3, wherein said at least one endocannabinoid and said N-acetylcysteine are in a weight ratio of 3:1 to 1:3.

6.  The composition of claim 5, wherein said at least one endocannabinoid and said N-acetylcysteine are in a weight ratio of 2:1 to 1:2.

7. The composition of claim 6, wherein said at least one endocannabinoid and said N-acetylcysteine are in a weight ratio of about 1:1.

8. The composition of any one claims 1-7, further comprising hibiscus and/or lactobacilli, preferably of species *L. rhamnosus*.

9. The composition of any one claims 1-8, in the form of a unitary dose comprising 100-1000 mg of said at least one endocannabinoid, 100-1000 mg of N-acetylcysteine, 500-5000 mg of mannose.

10. The composition of any one claims 1-9, in the form of a unitary dose comprising:

| | |
|---|---|
| - palmitoylethanolamide | 600 mg |
| - N-acetylcysteine | 600 mg |
| - D-mannose | 3000 mg |

or

| | |
|---|---|
| - palmitoylethanolamide | 300 mg |
| - N-acetylcysteine | 300 mg |
| - D-mannose | 1500 mg |

or

| | |
|---|---|
| - palmitoylethanolamide | 600 mg |
| - N-acetylcysteine | 600 mg |
| - D-mannose | 3000 mg |
| - hibiscus | 400 mg |

or

| | |
|---|---|
| - palmitoylethanolamide | 300 mg |
| - N-acetylcysteine | 300 mg |
| - D-mannose | 1500 mg |
| - hibiscus | 200 mg |

or

| | |
|---|---|
| - palmitoylethanolamide | 600 mg |
| - N-acetylcysteine | 600 mg |
| - D-mannose | 3000 mg |
| -*L. rhamnosus* LR04 | $2 \times 10^9$ UFC |

or

| | |
|---|---|
| - palmitoylethanolamide | 300 mg |
| - N-acetylcysteine | 300 mg |
| - D-mannose | 1500 mg |
| - *L. rhamnosus* LR04 | $1 \times 10^9$ UFC |

or

| - palmitoylethanolamide | 600 mg |
|---|---|
| - N-acetylcysteine | 600 mg |
| - D-mannose | 3000 mg |
| - hibiscus | 400 mg |
| - *L. rhamnosus* LR04 | 2x $10^9$ UFC |

or

| - palmitoylethanolamide | 300 mg |
|---|---|
| - N-acetylcysteine | 300 mg |
| - D-mannose | 1500 mg |
| - hibiscus | 200 mg |
| - *L. rhamnosus* LR04 | 1x $10^9$ UFC |

11. Pharmaceutical product comprising the composition of claims 1-10 and at least one antibiotic.

12. The composition of any one claims 1-10 or the pharmaceutical product of claim 11, for use in the treatment of urinary tract infections and/or of associated disorders selected from acute or chronic inflammation of the urinary tract, in particular cystitis and urethritis, and the symptoms thereof.

**Patentansprüche**

1. Zusammensetzung, umfassend D-Mannose, N-Acetylcystein und zumindest ein Endocannabinoid, wobei das zumindest eine Endocannabinoid Palmitoylethanolamid ist.

2. Zusammensetzung nach Anspruch 1, wobei die D-Mannose in einem Gewichtsverhältnis von 1:1 bis 20:1 in Bezug auf das zumindest eine Endocannabinoid oder auf das N-Acetylcystein ist.

3. Zusammensetzung nach Anspruch 2, wobei die Mannose in einem Gewichtsverhältnis von 1:1 bis 15:1 in Bezug auf das zumindest eine Endocannabinoid oder auf das N-Acetylcystein ist.

4. Zusammensetzung nach Anspruch 3, wobei die D-Mannose in einem Gewichtsverhältnis von 3:1 bis 10:1 in Bezug auf das zumindest eine Endocannabinoid oder auf das N-Acetylcystein ist.

5. Zusammensetzung nach einem der Ansprüche 1-3, wobei das zumindest eine Endocannabinoid und das N-Acetylcystein in einem Gewichtsverhältnis von 3:1 bis 1:3 sind.

6. Zusammensetzung nach Anspruch 5, wobei das zumindest eine Endocannabinoid und das N-Acetylcystein in einem Gewichtsverhältnis von 2:1 bis 1:2 sind.

7. Zusammensetzung nach Anspruch 6, wobei das zumindest eine Endocannabinoid und das N-Acetylcystein in einem Gewichtsverhältnis von etwa 1:1 sind.

8. Zusammensetzung nach einem der Ansprüche 1-7, ferner umfassend Hibiskus und/oder Lactobacillen, bevorzugt der Spezies *L. rhamnosus.*

9. Zusammensetzung nach einem der Ansprüche 1-8 in der Form einer Einheitsdosis, umfassend 100-1000 mg des zumindest einen Endocannabinoids, 100-1000 mg N-Acetylcystein, 500-5000 mg Mannose.

10. Zusammensetzung nach einem der Ansprüche 1-9 in der Form einer Einheitsdosis, umfassend:

| - Palmitoylethanolamid | 600 mg |
|---|---|
| - N-Acetylcystein | 600 mg |

(continued)

| | |
|---|---|
| - D-Mannose | 3000 mg |

oder

| | |
|---|---|
| - Palmitoylethanolamid | 300 mg |
| - N-Acetylcystein | 300 mg |
| - D-Mannose | 1500 mg |

oder

| | |
|---|---|
| - Palmitoylethanolamid | 600 mg |
| - N-Acetylcystein | 600 mg |
| - D-Mannose | 3000 mg |
| - Hibiskus | 400 mg |

oder

| | |
|---|---|
| - Palmitoylethanolamid | 300 mg |
| - N-Acetylcystein | 300 mg |
| - D-Mannose | 1500 mg |
| - Hibiskus | 200 mg |

oder

| | |
|---|---|
| - Palmitoylethanolamid | 600 mg |
| - N-Acetylcystein | 600 mg |
| - D-Mannose | 3000 mg |
| - *L. rhamnosus* LR04 | 2x $10^9$ UFC |

oder

| | |
|---|---|
| - Palmitoylethanolamid | 300 mg |
| - N-Acetylcystein | 300 mg |
| - D-Mannose | 1500 mg |
| - *L. rhamnosus* LR04 | 1x $10^9$ UFC |

oder

| | |
|---|---|
| - Palmitoylethanolamid | 600 mg |
| - N-Acetylcystein | 600 mg |
| - D-Mannose | 3000 mg |
| - Hibiskus | 400 mg |
| - *L. rhamnosus* LR04 | 2x $10^9$ UFC |

oder

| | |
|---|---|
| - Palmitoylethanolamid | 300 mg |
| - N-Acetylcystein | 300 mg |

(continued)

| | |
|---|---|
| - D-Mannose | 1500 mg |
| - Hibiskus | 200 mg |
| - *L. rhamnosus* LR04 | 1x $10^9$ UFC |

11. Pharmazeutisches Produkt, umfassend die Zusammensetzung nach Anspruch 1-10 und zumindest ein Antibiotikum.

12. Zusammensetzung nach einem der Ansprüche 1-10 oder pharmazeutisches Produkt nach Anspruch 11 zur Verwendung bei der Behandlung von Harnwegsinfektionen und/oder von assoziierten Störungen ausgewählt aus akuter oder chronischer Entzündung der Harnwege, insbesondere Zystitis und Urethritis, und den Symptomen davon.

**Revendications**

1. Composition comprenant du D-mannose, de la N-acétylcystéine et au moins un endocannabinoïde, ledit au moins un endocannabinoïde étant le palmitoyléthanolamide.

2. Composition de la revendication 1, dans laquelle ledit D-mannose est présent dans un rapport pondéral de 1:1 à 20:1 par rapport audit au moins un endocannabinoïde ou à ladite N-acétylcystéine.

3. Composition de la revendication 2, dans laquelle ledit mannose est présent dans un rapport pondéral de 1:1 à 15:1 par rapport audit au moins un endocannabinoïde ou à ladite N-acétylcystéine.

4. Composition de la revendication 3, dans laquelle ledit D-mannose est présent dans un rapport pondéral de 3:1 à 10:1 par rapport audit au moins un endocannabinoïde ou à ladite N-acétylcystéine.

5. Composition de l'une quelconque des revendications 1 à 3, dans laquelle ledit au moins un endocannabinoïde et ladite N-acétylcystéine sont présents dans un rapport pondéral de 3:1 à 1:3.

6. Composition de la revendication 5, dans laquelle ledit au moins un endocannabinoïde et ladite N-acétylcystéine sont présents dans un rapport pondéral de 2:1 à 1:2.

7. Composition de la revendication 6, dans laquelle ledit au moins un endocannabinoïde et ladite N-acétylcystéine sont présents dans un rapport pondéral d'environ 1:1.

8. Composition de l'une quelconque des revendications 1 à 7, comprenant en outre de l'hibiscus et/ou des lactobacilles, de préférence de l'espèce *L. rhamnosus.*

9. Composition de l'une quelconque des revendications 1 à 8, sous la forme d'une dose unitaire comprenant 100 à 1000 mg dudit au moins un endocannabinoïde, 100 à 1000 mg de N-acétylcystéine, 500 à 5000 mg de mannose.

10. Composition de l'une quelconque des revendications 1 à 9, sous la forme d'une dose unitaire comprenant :

| | |
|---|---|
| - palmitoyléthanolamide | 600 mg |
| - N-acétylcystéine | 600 mg |
| - D-mannose | 3000 mg |

ou

| | |
|---|---|
| - palmitoyléthanolamide | 300 mg |
| - N-acétylcystéine | 300 mg |
| - D-mannose | 1500 mg |

ou

| | |
|---|---|
| - palmitoyléthanolamide | 600 mg |
| - N-acétylcystéine | 600 mg |
| - D-mannose | 3000 mg |
| - hibiscus | 400 mg |

ou

| | |
|---|---|
| - palmitoyléthanolamide | 300 mg |
| - N-acétylcystéine | 300 mg |
| - D-mannose | 1500 mg |
| - hibiscus | 200 mg |

ou

| | |
|---|---|
| - palmitoyléthanolamide | 600 mg |
| - N-acétylcystéine | 600 mg |
| - D-mannose | 3000 mg |
| - *L. rhamnosus* LR04 | $2 \times 10^9$ UFC |

ou

| | |
|---|---|
| - palmitoyléthanolamide | 300 mg |
| - N-acétylcystéine | 300 mg |
| - D-mannose | 1500 mg |
| - *L. rhamnosus* LR04 | $1 \times 10^9$ UFC |

ou

| | |
|---|---|
| - palmitoyléthanolamide | 600 mg |
| - N-acétylcystéine | 600 mg |
| - D-mannose | 3000 mg |
| - hibiscus | 400 mg |
| - *L. rhamnosus* LR04 | $2 \times 10^9$ UFC |

ou

| | |
|---|---|
| - palmitoyléthanolamide | 300 mg |
| - N-acétylcystéine | 300 mg |
| - D-mannose | 1500 mg |
| - hibiscus | 200 mg |
| - *L. rhamnosus* LR04 | $1 \times 10^9$ UFC. |

11. Produit pharmaceutique comprenant la composition des revendications 1 à 10 et au moins un antibiotique.

12. Composition de l'une quelconque des revendications 1 à 10 ou produit pharmaceutique de la revendication 11, destiné(e) à être utilisé(e) dans le traitement d'infections des voies urinaires et/ou de troubles associés choisis parmi une inflammation aiguë ou chronique des voies urinaires, en particulier une cystite et une urétrite, et les symptômes de ceux-ci.

Fig. 1

Fig. 2

3,13 g/L      1,56 g/L      0,78 g/L      0,39 g/L

321 µg/ml      10 µg/ml      5 µg/ml      2,5 µg/ml

Fig. 3

3,13 g/L      1,56 g/L      0,78 g/L      0,39 g/L

78 µg/ml      39 µg/ml      2,44 µg/ml      1,22 µg/ml

Fig. 4

Fig. 5

Fig. 6

Fig. 7

Fig. 8

Fig. 9

Fig. 10

Fig. 11

Fig. 12

12,5 g/L    6,25 g/L    0,78 g/L    0,39 g/L

1

2

65 g/L    32,5g/L    16,25 g/L    8,13 g/L

Fig. 13

12,5 g/L    6,25 g/L    0,78 g/L    0,39 g/L

1

2

65 g/L    32,5g/L    16,25 g/L    8,13 g/L

Fig. 14

Fig. 15

Fig. 16

Fig. 17

Fig. 18

Fig. 19

| Biofilm untreated | Composition Ex. 1 3,13 g/l | D-mannose + NAC 2,23 g/l ; 0,45 g/l | PEA 0,45 g/l |
|---|---|---|---|

Fig. 20

| Biofilm untreated | Composition Ex. 1 1,56 g/l | D-mannose + NAC 1,12 g/l ; 0,22 g/l | PEA 0,22 g/l |
|---|---|---|---|

Fig. 21

Fig. 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015173715 A1

**Non-patent literature cited in the description**

- **MARK FELDMAN et al.** *Antimicrobial potential of endocannabinoid and endocannabinoid-like compounds against methicillin-resistant Staphylococcus aureus*, 2018

- **SARA SOTO et al.** *Importance of Biofilms in Urinary Tract Infections: New Therapeutic Approaches*, 2014